# EUROPÄISCHE PATENTSCHRIFT

(11) **EP 2 164 139 B1**
(45) Veröffentlichungstag und Bekanntmachung des Hinweises auf die Patenterteilung: **11.11.2015**
(21) Anmeldenummer: 09165799.9
(22) Anmeldetag: 17.07.2009
(51) Int. Cl.: H01R 13/52, H01R 13/74

(54) **Medizintechnisches Gerät und medizintechnische Geräteanordnung**
Medical device and medical device assembly
Appareil médical et agencement d'appareils médicaux

(30) Priorität: 15.09.2008 DE 102008047339
(43) Veröffentlichungstag der Anmeldung: 17.03.2010
(73) Patentinhaber: Olympus Winter & Ibe GmbH, 22045 Hamburg (DE)
(72) Erfinder: Hörlle, Andreas, 14163 Berlin (DE); Sprenger, Christopher, 10961 Berlin (DE); Roggan, Dr., André, 12163 Berlin (DE); Kühne, Wolfgang, 16567 Schönfließ (DE); Schiddel, Stefan, 14480 Potsdam (DE); Fischer, Uwe, 10439 Berlin (DE); Strauß, Timo, 10711 Berlin (DE)
(74) Vertreter: Eisenführ Speiser

(56) Entgegenhaltungen:
- WO-A1-2006/034513
- JP-A- 2001 104 334
- JP-A- 2005 024 707
- US-A- 3 794 956
- US-A- 6 046 405
- US-B1- 6 435 903
- US-S1- D 456 357

## Beschreibung

Die Erfindung betrifft ein medizintechnisches Gerät zur Behandlung des menschlichen oder tierischen Körpers, mit wenigstens einem elektrischen Steckverbinder, der mit einem komplementären Gegensteckverbinder eines weiteren medizintechnischen Gerätes verbindbar ausgebildet ist und der in einer Aktivposition an der Oberseite des Geräts, nach oben vorstehend angeordnet ist.

Die vielfältigen Anwendungsmöglichkeiten der Medizintechnik haben bereits seit vielen Jahren zu einer Vielzahl von spezialisierten Endgeräten geführt, wie zum Beispiel HochfrequenzGeneratoren, Ultraschall-Generatoren oder Argonplasmaeinheiten. Um dem Arzt für verschiedene Anwendungen stets ein optimales Gerät zur Verfügung zu stellen, sind multifunktionale Geräte oder Geräteanordnungen entwickelt worden. Diese Geräte oder Geräteanordnungen werden im einfachsten Fall durch das elektrische und mechanische Verbinden zweier oder mehrerer Geräte erreicht. Ein bekanntes Beispiel ist die Verbindung einer Argonplasmaeinheit mit einem Hochfrequenz-, kurz HF-Generator. Dabei wird ein Hochfrequenzstrom über eine Steckverbindung in die Argonplasmaeinheit geleitet, um ein Argonplasma/HF-Handinstrument an der Argonplasmaeinheit anschließen zu können.

Die Kabelverbindung für die Übertragung des HF-Signals zwischen den Geräten sollte möglichst kurz gehalten werden. Mit steigender Länge nehmen beim Leiten von hochfrequenter Wechselspannung die elektrischen Leckströme oder Ableitströme zu und die elektromagnetische Verträglichkeit ab. Daher ist die HF-Steckverbindung zwischen den üblicherweise übereinander stehenden Geräten vorzugsweise in der Nähe der Ausgangsbuchsen der Handinstrumente angeordnet.

Das Dokument JP 2001-104334A offenbart ein medizintechnisches Gerät zur Behandlung des menschlichen oder tierischen Körpers, mit wenigstens einem optischen Steckverbinder, der mit einem komplementären Gegensteckverbinder eines weiteren medizintechnischen Gerätes verbindbar ausgebildet ist und der in einer Aktivposition an der Oberseite des Geräts, nach oben vorstehend angeordnet ist.

Bei allen bekannten Geräteanordnungen besteht das Problem, dass die Geräte nicht oder zumindest nicht beide als Einzelgerät verwendet werden können. Wenigstens bei einem der Geräte liegen im entkoppelten Zustand elektrische Kontakte offen, so dass bestehende Medizingerätesicherheitsnormen, beispielsweise in Bezug des Spritzwasserschutzes, nicht eingehalten werden können. Weiterhin erfüllen die entkoppelten Einzelgeräte meist nicht die heute üblichen ästhetischen Anforderungen.

Der vorliegenden Erfindung liegt daher die Aufgabe zugrunde ein medizintechnisches Gerät zu Verfügung zu stellen, das sowohl als Einzelgerät als auch als in Verbindung mit einem zweiten Gerät als Geräteanordnung verwendet werden kann und dabei bestehende Sicherheits- und Ästhetikanforderungen erfüllt.

Diese Aufgabe wird erfindungsgemäß von dem oben genannten medizintechnischen Gerät dadurch erfüllt, dass der Steckverbinder von der Aktivposition in eine Passivposition bewegbar ausgebildet ist, wobei in der Passivposition der Steckverbinder von der Oberseite des Geräts entfernt und die Oberseite flüssigkeitsdicht verschlossen ist.

Die erfindungsgemäße Lösung hat den Vorteil, dass das medizintechnische Gerät auf sehr einfache Weise zwischen Einzelbetrieb oder Kombinationsbetrieb umgebaut werden kann. Die Einsatzmöglichkeiten des erfindungsgemäßen Gerätes werden so erheblich vergrößert, weil es nun sowohl als Einzelgerät als auch als Kombinationsgerät verwendet werden kann.

Die erfindungsgemäße Lösung kann durch weitere vorteilhafte Ausgestaltungen ergänzt werden. Einige solcher Ausgestaltungen sind im Folgenden beschrieben.

So kann das medizintechnische Gerät wenigstens eine Lifteinheit aufweisen, durch die der Steckverbinder mit dem übrigen Gerät beweglich verbunden ist. Dies hat den Vorteil, dass der Steckverbinder und damit das medizintechnische Gerät besonders einfach und schnell zwischen Aktiv- und Passivposition umgebaut werden kann. Insbesondere kann die Lifteinheit ein Schubgelenk aufweisen, mittels dem der Steckverbinder im Wesentlichen linear von der Aktivposition in die Passivposition bewegbar ist. Zum Steckverbinder führen ein oder mehrere Kabel, die mit elektrischen Kontakten des Steckverbinders verbunden sind. Es hat sich gezeigt, dass die Linearbewegung des Schubgelenks besonders gut geeignet ist, um das Kabel schonend zum Steckverbinder zu führen. Alternativ kann die Lifteinheit selbstverständlich auch ein Drehgelenk oder jede andere Gelenkform aufweisen. Auch Ausführungsformen mit einem Verbindungsdraht bzw. -kette oder auch dem Kabel als Verbindungselement sind möglich.

Um auf einfache Weise den Spritzwasserschutz bei der Verwendung als Einzelgerät zu gewährleisten, kann das Gerät wenigstens ein Abdeckmittel umfassen, das die Oberseite des Geräts in der Passivposition im Wesentlichen flüssigkeitsdicht verschließt. Ferner kann der Steckverbinder mit dem Abdeckmittel verbunden und in der Passivposition im Innern des Gerätes angeordnet sein. So dient das Abdeckmittel, beispielsweise eine Abdeckplatte, gleichzeitig als Halterung für den Steckverbinder.

In einer weiteren vorteilhaften Ausgestaltung kann das Gerät wenigstens ein Antriebsmittel aufweisen, das den Steckverbinder von der Passivposition in die Richtung der Aktivposition drückt. Dies hat den Vorteil, dass sich der Steckverbinder selbsttätig von der schlecht zugänglichen Passivposition im Innern des medizintechnischen Geräts in die Aktivposition bewegt. Als Antriebsmittel kann beispielsweise ein Federelement wie eine Schraubendruckfeder verwendet werden.

Die Erfindung umfasst ferner auch eine medizintechnische Geräteanordnung zur Behandlung des menschlichen oder tierischen Körpers, mit wenigstens zwei medizintechnischen Geräten, die über eine Steckverbinderanordnung miteinander elektrisch und mechanisch verbunden sind, wobei die Steckverbinderanordnung einen mit dem ersten Gerät verbundenen Steckverbinder und einen mit dem zweiten Gerät verbundenen, zum Steckverbinder komplementär ausgebildeten Gegensteckverbinder umfasst. Um beide Geräte separat verwenden zu können, ist das erste medizintechnische Gerät nach einer der zuvor genannten Ausführungsformen ausgebildet.

In einer besonders vorteilhaften Ausgestaltung der Geräteanordnung können das erste Gerät ein Ultraschallgenerator und das zweite Gerät ein HF-Generator sein. Für Ultraschallgeneratoren und HF-Generatoren gibt es viele Anwendungen als Einzelgeräte und auch als Kombinationsgerät, so dass sich sehr viele Einsatzmöglichkeiten der Geräteanordnung ergeben.

Um kombinierte Ultraschall-HF-Instrumente anzuschließen, kann die Geräteanordnung so ausgebildet sein, dass im Betrieb am Ultraschallgenerator sowohl ein Hochfrequenzstromsignal als auch ein Signal für ein Ultraschallinstrument gleichzeitig abgreifbar sind. Kombinierte Ultraschall-HF-Instrumente erzeugen beispielsweise Ultraschallenergie zum Gewebeschneiden und nutzen HF-Energie, um das Gewebe vor dem Schneiden zu Koagulieren.

Im Folgenden wird die Erfindung anhand der in den Zeichnungen dargestellten beispielhaften Ausführungsformen erläutert. Die unterschiedlichen Merkmale können, wie auch bei den oben beschriebenen Ausführungsformen, beliebig miteinander kombiniert werden.

Es zeigen:
- Fig. 1: eine schematische Darstellung einer beispielhaften Ausführungsform einer erfindungsgemäßen Geräteanordnung;
- Fig. 2: eine schematische Darstellung einer ersten beispielhaften Ausführungsform eines erfindungsgemäßen medizintechnischen Geräts von oben;
- Fig. 3: eine schematische Darstellung des medizintechnischen Geräts aus Fig. 2 von unten;
- Fig. 4: eine schematische Darstellung einer beispielhaften Ausführungsform eines Gegensteckers der Geräteanordnung aus Fig. 1;
- Fig. 5: eine schematische Darstellung des Gegensteckverbinders aus Fig. 4 von oben;
- Fig. 6: eine schematische Darstellung einer weiteren beispielhaften Ausführungsform eines erfindungsgemäßen medizintechnischen Geräts in einer Aktivposition;
- Fig. 7: eine schematische Darstellung des medizintechnischen Geräts aus Fig. 6 in einer Passivposition;
- Fig. 8: eine schematische Darstellung einer beispielhaften Ausführungsform eines Gegensteckers für eine Geräteanordnung mit dem Gerät aus Fig. 6 und 7;
- Fig. 9: eine schematische Darstellung einer weiteren beispielhaften Ausführungsform eines erfindungsgemäßen medizintechnischen Geräts in einer Aktivposition;
- Fig. 10: eine schematische Darstellung des medizintechnischen Geräts aus Fig. 9 in einer Passivposition;
- Fig. 11: eine schematische Darstellung eines Steckverbinders einer weiteren beispielhaften Ausführungsform eines erfindungsgemäßen medizintechnischen Geräts;
- Fig. 12: eine schematische Darstellung der erfindungsgemäßen Geräteanordnung aus Fig. 1 mit verbundenen Geräten.

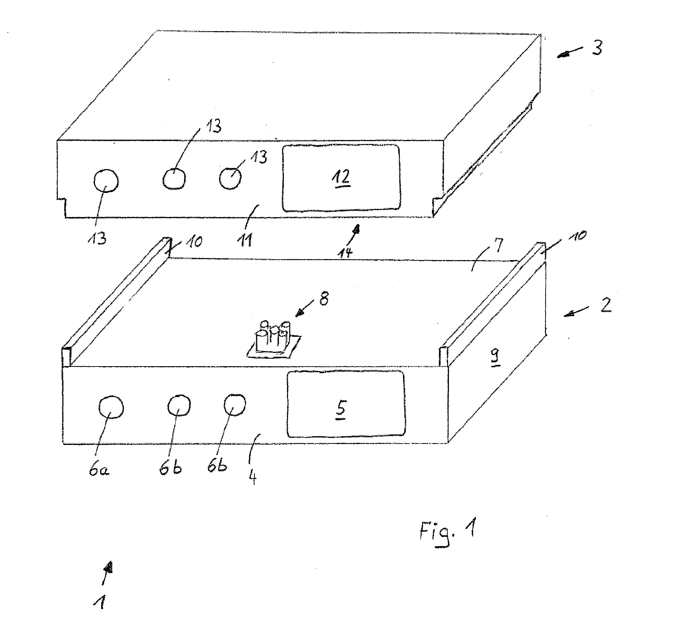

Eine medizintechnische Geräteanordnung 1 in Fig. 1 umfasst einen Ultraschallgenerator 2 und einen HF-Generator 3.

Der Ultraschallgenerator 2 weist an der Frontseite 4 ein Touch-Screen Bedienfeld 5 sowie Ausgangsbuchsen 6a, 6b auf. An seinem oberseitigen Gehäusedeckel 7, in der Nähe der Frontseite 4, weist der Ultraschallgenerator 2 einen nach oben vorstehenden Steckverbinder 8 auf. Ebenfalls nach oben vorstehend, jedoch seitlich in Verlängerung der Seitenwände 9 sind zwei Halteleisten 10 angebracht.

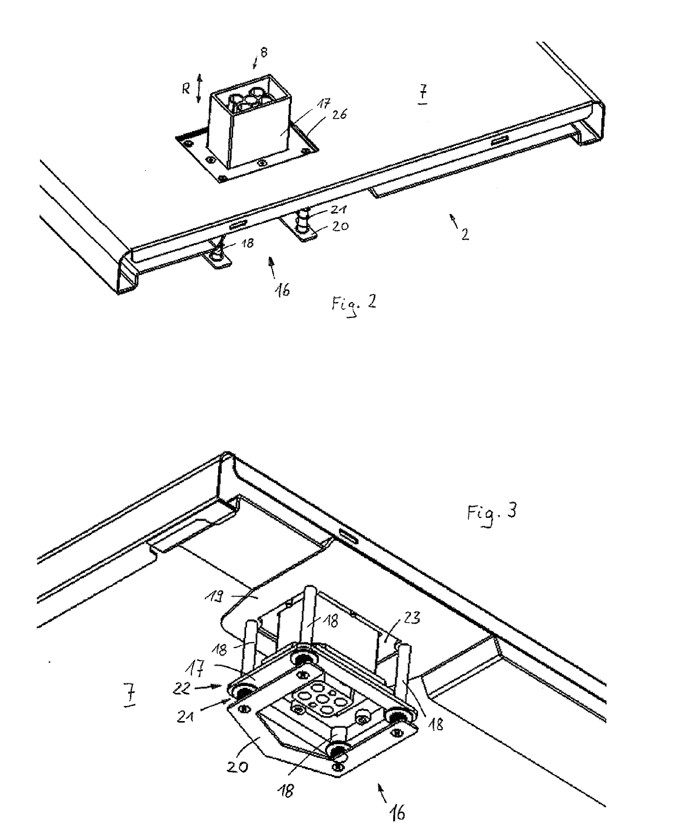

Die Ausgangsbuchsen 6a sind zum Anschließen von chirurgischen Ultraschallinstrumenten und die Ausgangsbuchsen 6b zum Anschließen von kombinierten HF/Ultraschallinstrumenten ausgebildet. Kombinierte HF/Ultraschallinstrumente können ein Ultraschallsignal erzeugen und weisen zusätzlich eine oder mehrere Elektroden zur Abgabe von HF-Energie an das zu behandelnde Gewebe auf. Da der Ultraschallgenerator 2 selbst keine HF-Energie für die kombinierten HF/Ultraschallinstrumente erzeugen kann, wird sie über den Steckverbinder 8 vom HF-Generator 3 in den Ultraschallgenerator 2 eingeleitet, wie es im Folgenden mit Bezug auf die Fig. 2 bis 11 noch näher beschrieben wird. Über das Touch-Screen Bedienfeld 5 kann der Benutzer sowohl für den Einzel- als auch den Kombinationsbetrieb nötige Einstellungen vornehmen. Die Halteleisten 10 arretieren den Ultraschallgenerator 2 zum HF-Generator 3 im kombinierten Zustand und können mit Befestigungsmitteln, wie z.B. Schrauben, an den Generatoren 2, 3 befestigt sein. Alternativ können die Halteleisten 10 beispielsweise auch in Verlängerung der Rückwand ausgebildet sein.

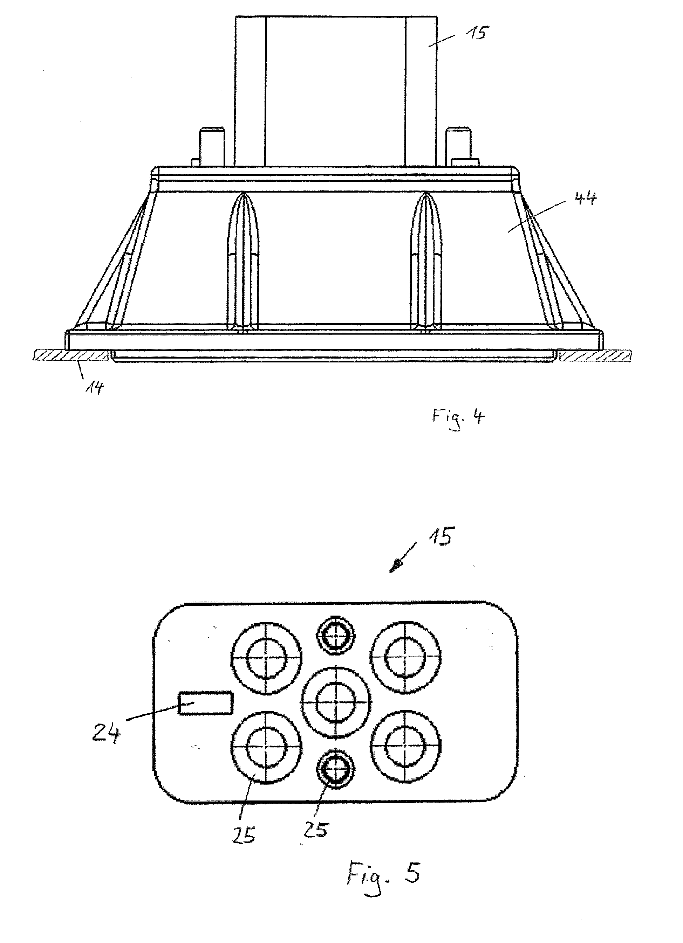

An der Frontseite 11 des HF-Generators 3 sind ähnlich wie beim Ultraschallgenerator 2 ebenfalls ein Touch-Screen Bedienfeld 12 und verschiedene Ausgangsbuchsen 13 angeordnet. Im Gehäuseboden 14 des HF-Generators 3 ist, in Fig. 1 nicht sichtbar, ein zum Steckverbinder 8 des Ultraschallgenerators 2 komplementär ausgebildeter Gegensteckverbinder 15 vorgesehen. Die Ausgestaltung des Gegensteckverbinders 15 ist in Fig. 4 dargestellt und wird im Folgenden noch näher beschrieben.

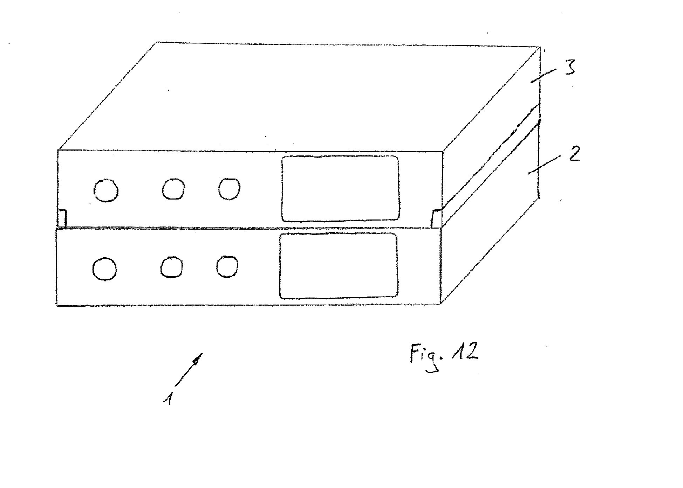

In Fig. 1 sind der Ultraschallgenerator 2 und der HF-Generator 3 in einer Position dargestellt, bevor sie verbunden werden. In Fig. 12 ist die Geräteanordnung 1 mit verbundenen Generatoren 2, 3 dargestellt, so wie sie im Kombinationsbetrieb angeordnet sind. Selbstverständlich können auch andere medizintechnische Geräte mit dem erfindungsgemäßen Steckverbinder verbunden werden können.

Der HF-Generator 3 überträgt im Kombinationsbetrieb HF-Energie über den Gegensteckverbinder 15, der mit dem Steckverbinder 8 gekoppelt ist, an den Ultraschallgenerator 2. Ansonsten weist der HF-Generator 3 auch alle Merkmale eines handelsüblichen HF-Generators auf, auf die hier nicht näher eingegangen wird.

Anhand der Fig. 2 und 3 wird nun eine erste Ausführungsform des erfindungsgemäßen medizintechnischen Gerätes 2 mit dem Steckverbinder 8 näher beschrieben. Der Übersichtlichkeit halber sind in den Fig. 2 und 3 die für den Steckverbinder 8 nicht relevante Teile, wie beispielsweise die Frontseite, des Ultraschallgenerators 2 weg gelassen.

Fig. 2 zeigt den Steckverbinder 8 in einer Aktivposition, in welcher der Steckverbinder 8 ausgefahren ist und über den Gehäusedeckel 7 nach oben vorsteht. In Fig. 3 ist der Steckverbinder 8 in einer Passivposition dargestellt, in der er ins Innere des Ultraschallgenerators 2 eingefahren ist, so dass der Steckverbinder nicht über den Gehäusedeckel 7 hervorsteht.

Der Steckverbinder 8 ist bei der Ausführungsform der Fig. 2 und 3 mit einer Lifteinheit 16 verbunden, die am Gehäusedeckel 7 befestigt ist. Lifteinheit 16 mit dem Steckverbinder 8 sind in einem rechteckigen Ausschnitt 26 des Gehäusedeckels 7 angeordnet. In den Fig. 2 und 3 sind die Lifteinheit 16 mit dem Gehäusedeckel 7 und der Steckverbinder 8 mit der Lifteinheit 16 verschraubt. Alternativ können sie aber auch verschweißt, genietet, geklebt oder in sonstiger Weise befestigt sein. Die Lifteinheit 16 weist einen rechteckigen Flanschrahmen 17 auf, mit dem der Steckverbinder 8 verbunden ist. Der Flanschrahmen 17 ist mit einer Linearführung in Form von vier Führungsstangen 18 beweglich gekoppelt. Die gleichlangen, parallel zueinander verlaufenden Führungsstangen 18 sind an den Enden jeweils zueinander fixiert. Die oberen Enden sind über ein Verstärkungsblech 19 mit dem Gehäusedeckel 7 gekoppelt. Die unteren Enden sind mit einen im Wesentlichen U-förmigen Fixierblech 20 verbunden. Um die Führungsstangen 18 herum, zwischen dem Flanschrahmen 17 und dem Fixierblech 20 sind Schraubendruckfedern 21 angeordnet.

Durch die Lifteinheit 16 ist der Steckverbinder 8 linear beweglich angeordnet. Der Flanschrahmen 17 bildet mit den Führungsstangen 18 ein Schubgelenk 22 aus, mittels dem der Steckverbinder 8 im Wesentlichen linear zwischen der Aktivposition in Fig. 2 in die Passivposition in Fig. 3 in einer Bewegungsrichtung R bewegbar ist. Die Bewegungsrichtung R verläuft im Wesentlichen rechtwinkelig zu Ebene des Gehäusedeckels 7. Um die Reibung zwischen den Führungsstangen 18 und dem Flanschrahmen 17 zu reduzieren, können beispielsweise Gleitbuchsen in den Flanschrahmen 17 eingesetzt sein.

Der Flanschrahmen 17 ist im Wesentlichen zweistufig aufgebaut und weist einen breiten unteren Flansch sowie einen schmalen oberen Flansch auf. Der breite untere Flansch ist mit den Führungsstangen 18 verbunden und befindet sich stets im Innern des Ultraschallgenerators 2. Der schmale obere Flansch ist mit dem Steckverbinder verbunden und steht in der Aktivposition über den Gehäusedeckel 7 vor. In der Mitte weist der der Flanschrahmen 17 einen Kabeldurchlass auf, durch den die Kabel (nicht dargestellt) zum Steckverbinder geleitet werden können.

In der Aktivposition in Fig. 2 kann die Lifteinheit 16 arretiert werden, um ein ungewolltes Versenken des Steckverbinders 8 beispielsweise beim Zusammensetzen der beiden Generatoren 2, 3 zu verhindern. Die Arretierung kann beispielsweise durch Verschrauben oder aber auch durch Rasten realisiert werden.

Die Schraubendruckfedern 21 sind als Antriebselement vorgesehen, das die Lifteinheit 16 und damit den Steckverbinder 8 stets in die Richtung der Aktivposition drückt. So ist es für den Bediener besonders einfach den Steckverbinder 8 von der Innen liegenden Passivposition, in welcher der Steckverbinder 8 schlecht zugänglich ist, in die Aktivposition zu bewegen.

Der Ultraschallgenerator 2 umfasst weiterhin eine Abdeckung 23, die den Gehäusedeckel 7 in der Passivposition im Wesentlichen flüssigkeitsdicht verschließt. Die Abdeckung 23 ist beispielsweise aus einem Metallblech hergestellt, das mit dem Gehäusedeckel 7 verschraubt wird. Um die Dichtwirkung zu verstärken kann zwischen der Abdeckung 23 und dem Gehäusedeckel 7 eine Dichtung angeordnet sein. Die Abdeckung 23 kann gleichzeitig auch als Arretierung dienen, welche die Lifteinheit 16 in der Passivposition hält. Alternativ kann selbstverständlich auch in der Passivposition eine Rastung oder ähnliches als Arretierung der Lifteinheit 16 vorgesehen sein.

Fig. 4 zeigt den im HF-Generator 3 eingebauten Gegensteckverbinder 15. Der Gegensteckverbinder 15 ist mit einer trichterförmigen Halterung 44 verbunden, die am Gehäuseboden 14 befestigt ist. Die trichterförmige Ausgestaltung der Halterung 44 erleichtert das Positionieren des Steckverbinders 8 zum Gegensteckverbinder 15 beim Koppeln der beiden Generatoren 2, 3. Der Gegensteckverbinder 15 mit der Halterung 44 bleibt beim Trennen oder Verbinden der beiden Generatoren 2, 3 unverändert. Die elektrischen Kontakte (nicht dargestellt) im Gegensteckverbinder 15 sind so angeordnet, dass sie bei getrennten Geräten 2, 3 vom Bediener nicht berührt werden können.

In Fig. 5 ist der Gegensteckverbinder 15 in einer Ansicht von unten gezeigt. Der Gegensteckverbinder 15 umfasst einen mechanischen Orientierungszapfen 24, um ein falsches Einstecken zu verhindern, und Bohrungen für Befestigungsschrauben. Ferner weist er mehrere Kontaktstifte 25 zur Übertragung verschiedener elektrischer Signale auf, wie beispielsweise HF-Signale für monopolare und bipolare Anwendungen sowie Signale zur Verbindungserkennung. Der Gegensteckverbinder 15 ist komplementär zum Steckverbinder 8 ausgebildet.

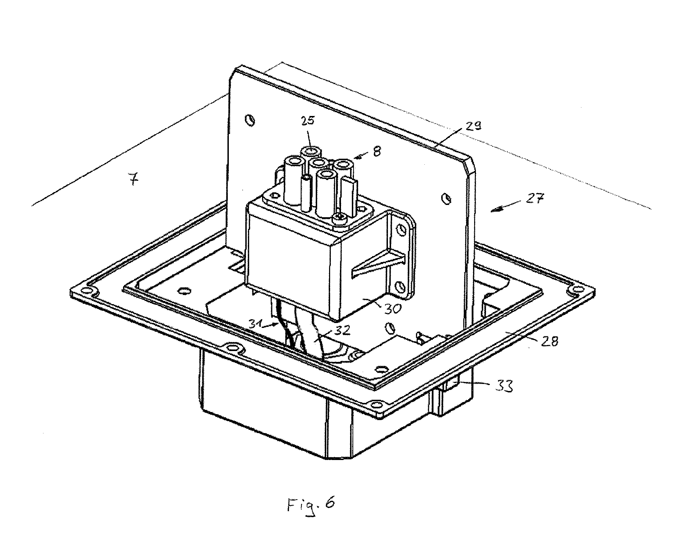

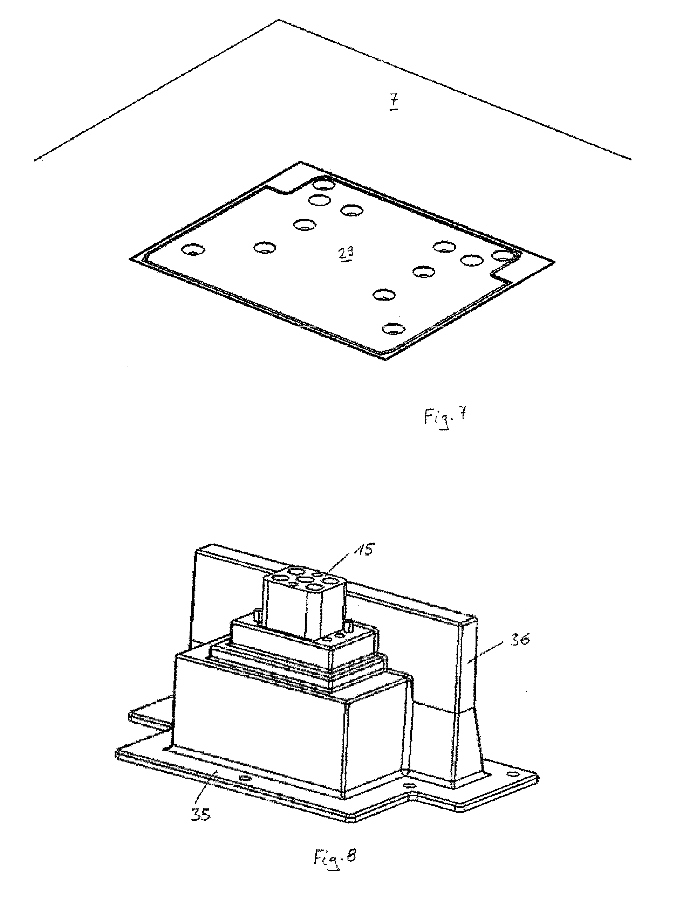

Eine weitere Ausführungsform des erfindungsgemäßen Gerätes 2 mit einer alternativen Lifteinheit 27 ist in den Fig. 6 und 7 dargestellt und wird im Folgenden beschrieben. Der Kürze halber wird nur auf die Unterschiede zu der oben beschriebenen Ausführungsform eingegangen. In Fig. 6 ist der Gehäusedeckel 7 der Übersichtlichkeit halber transparent dargestellt. In Fig. 6 ist der Steckverbinder 8 mit einer alternativen Lifteinheit 27 in der Aktivposition, in Fig. 7 in der Passivposition dargestellt.

Die Lifteinheit 27 der Fig. 6 und 7 umfasst eine Aufnahmeschale 28, eine Wendeplatte 29 und ein Zwischenstück 30, an dem der Steckverbinder 8 befestigt ist. Ferner weist die Lifteinheit 27 einen Verbindungsdraht 31 als mechanische, bewegliche Verbindung zwischen der Wendeplatte 29 und der Aufnahmeschale 28 auf. Ein elektrisches Anschlusskabel 32 verbindet die Kontakte (nicht dargestellt) des Steckverbinders 8 mit den entsprechenden Kontakten (nicht dargestellt) im Innern des Ultraschallgenerators 2. Das Anschlusskabel 32 ist länger als der Verbindungsdraht 31, um Zugkräfte auf dem Anschlusskabel 32 zu vermeiden. Die Aufnahmeschale 28 weist eine Aufnahmesenke 33 auf, in welcher die Wendeplatte 29 in der Aktivposition in Fig. 5 steckt. Die Kontaktstifte 25 des Steckverbinders 8 verlaufen im Wesentlichen parallel zur ebenen Wendeplatte 29, die im Wesentlichen rechtwinkelig zum Gehäusedeckel 7 in der Aufnahmesenke 33 steckt. So steht der Steckverbinder 8 in der Aktivposition über den Gehäusedeckel 7 vor.

Um den Steckverbinder 8 in die Passivposition zu bewegen, wird die Wendeplatte 29 aus der Aufnahmesenke 33 entfernt und um 90° gedreht auf die Aufnahmeschale 28 gelegt, so dass sich der Steckverbinder 8 im Innern der Aufnahmesenke 33 befindet. Die Passivposition ist in Fig. 7 mit Sicht von oben auf den Ultraschallgenerator 2 dargestellt. Die Wendeplatte 29 kann in der Passivposition mit Befestigungsmitteln, wie beispielsweise Schrauben (nicht dargestellt), fixiert werden.

Fig. 8 zeigt eine für die Ausführungsform der Fig. 6 und 7 angepasste Halterung 35 für den Gegensteckverbinder 15. Im Unterschied zur Halterung 44 in Fig. 4 weist die Halterung 35 eine Formsenke 36 auf, in die die Wendeplatte 29 beim Verbinden der Geräte 2, 3 von unten eintauchen kann.

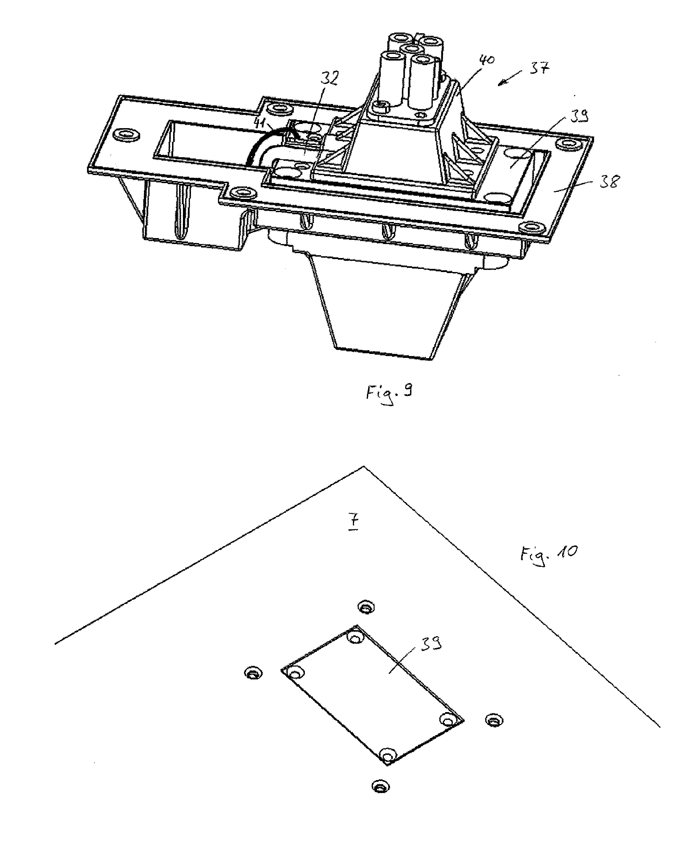

Eine weitere Ausführungsform des erfindungsgemäßen Gerätes 2 mit einer alternativen Lifteinheit 37 ist in den Fig. 9 und 10 dargestellt und wird im Folgenden beschrieben. Der Kürze halber wird nur auf die Unterschiede zu den oben beschriebenen Ausführungsformen eingegangen. In Fig. 9 ist der Gehäusedeckel 7 der Ultraschallgenerators 2 nicht dargestellt. In Fig. 9 ist der Steckverbinder 8 mit einer alternativen Lifteinheit 37 in der Aktivposition, in Fig. 10 in der Passivposition dargestellt.

Wie die Ausführungsform in Fig. 6, umfasst auch die Lifteinheit 37 der Ausführungsform in Fig. 9 und 10 eine Aufnahmeschale 38, eine Wendeplatte 39 und ein Zwischenstück 40, an dem der Steckverbinder 8 befestigt ist. Ein Verbindungsdraht 41 verbindet auch hier die Wendeplatte 39 mit der Aufnahmeschale 38. Allerdings muss die Wendeplatte 39 um 180° gedreht werden, um die Lifteinheit 37 von der Aktivposition in Fig. 9 in die Passivposition in Fig. 10 zu bewegen. Auch in der Passivposition in Fig. 10 kann die Wendeplatte 39 beispielsweise mit Befestigungsschrauben (nicht dargestellt) gesichert werden. Für diese Ausführungsform kann ebenfalls der Gegensteckverbinder 15 mit der Halterung 44 aus Fig. 4 verwendet werden.

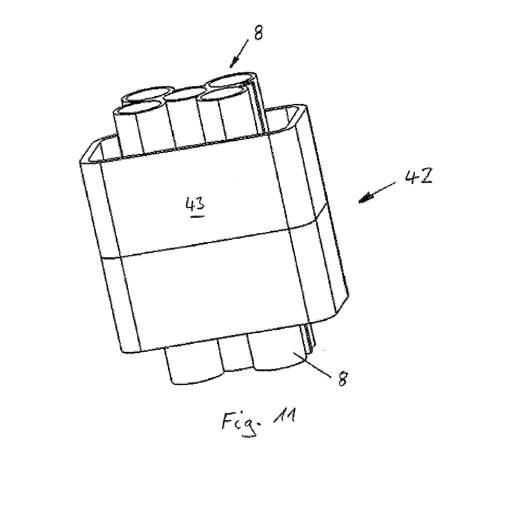

Eine weitere Ausführungsform der Erfindung ist in Fig. 11 dargestellt, Fig. 11 zeigt einen Doppelsteckverbinder 42, bei dem an jedem Ende ein Steckverbinder 8 angeordnet ist. Die Steckverbinder 8 sind in einem Gehäuse 43 fixiert und deren Kontakte (nicht dargestellt) im Innern des Gehäuses jeweils miteinander verbunden. Für die Verwendung des Doppelsteckverbinders 42 sind sowohl der Ultraschallgenerator 2 als auch der HF-Generator 3 mit der Halterung 44 und dem Gegensteckverbinder 15 aus Fig. 4 ausgestattet. In der Aktivposition ist der Doppelsteckverbinder 42 auf den Gegensteckverbinder 15 des Ultraschallgenerators 2 aufgesteckt. So kann der Ultraschallgenerator 2 mit dem HF-Generator 3 verbunden werden. Für die Passivposition wird der Doppelsteckverbinder 42 entfernt und eine Abdeckplatte verschließt den Gehäusedeckel 7 flüssigkeitsdicht.

## Patentansprüche

1. Medizintechnisches Gerät zur Behandlung des menschlichen oder tierischen Körpers, mit wenigstens einem elektrischen Steckverbinder, der mit einem komplementären Gegensteckverbinder eines weiteren medizintechnischen Gerätes verbindbar ausgebildet ist und der in einer Aktivposition an der Oberseite des Geräts, nach oben vorstehend angeordnet ist, wobei der Steckverbinder von der Aktivposition in eine Passivposition bewegbar ausgebildet ist, wobei in der Passivposition der Steckverbinder von der Oberseite des Geräts entfernt und die Oberseite flüssigkeitsdicht verschlossen ist.

2. Gerät nach Anspruch 1, **dadurch gekennzeichnet, dass** das Gerät wenigstens eine Lifteinheit aufweist, durch die der Steckverbinder mit dem übrigen Gerät beweglich verbunden ist.

3. Gerät nach Anspruch 2, **dadurch gekennzeichnet, dass** die Lifteinheit ein Schubgelenk aufweist, durch das der Steckverbinder im Wesentlichen linear von der Aktivposition in die Passivposition bewegbar ausgebildet ist.

4. Gerät nach einem der oben genannten Ansprüche, **dadurch gekennzeichnet, dass** das Gerät wenigstens ein Abdeckmittel umfasst, das die Oberseite des Geräts in der Passivposition im Wesentlichen flüssigkeitsdicht verschließt.

5. Gerät nach Anspruch 4, **dadurch gekennzeichnet, dass** der Steckverbinder mit dem Abdeckmittel verbunden ist und in der Passivposition im Innern des Gerätes angeordnet ist.

6. Gerät nach einem der oben genannten Ansprüche, **dadurch gekennzeichnet, dass** das Gerät wenigstens ein Antriebsmittel aufweist, das den Steckverbinder von der Passivposition in die Richtung der Aktivposition drückt.

7. Medizintechnische Geräteanordnung zur Behandlung des menschlichen oder tierischen Körpers, mit wenigstens zwei medizintechnischen Geräten, die über eine Steckverbinderanordnung miteinander elektrisch und mechanisch verbunden sind, wobei die Steckverbinderanordnung einen mit dem ersten Gerät verbundenen Steckverbinder und einen mit dem zweiten Gerät verbundenen, zum Steckverbinder komplementär ausgebildeten Gegensteckverbinder umfasst, **dadurch gekennzeichnet, dass** das erste medizintechnische Gerät nach einem der oben genannten Ansprüche ausgebildet ist.

8. Geräteanordnung nach Anspruch 7, **dadurch gekennzeichnet, dass** das erste Gerät ein Ultraschallgenerator und das zweite Gerät ein HF-Generator ist.

9. Geräteanordnung nach Anspruch 8, **dadurch gekennzeichnet, dass** die Geräteanordnung so ausgebildet ist, dass im Betrieb am Ultraschallgenerator sowohl ein Hochfrequenzstromsignal als auch ein Signal für ein Ultraschallinstrument gleichzeitig abgreifbar sind.

## Claims

1. Medical device for the treatment of human or animal bodies, with at least one electrical connector, which is designed connectable to a complementary mating connector of another medical device and which is arranged projecting upwards in an active position at the top of the device, whereby the connector is designed to be moved from the active position into a passive position, whereby the connector is removed from the top of the device and the top is closed in a fluid-tight way in the passive position.

2. Device according to claim 1, **characterised in that** the device comprises at least one lift unit, via which the connector is moveably connected with the rest of the device.

3. Device according to claim 2, **characterised in that** the lift unit comprises a slider element, with which the connector is designed to be moved substantially linear from the active position to the passive position.

4. Device according to one of the above mentioned claims, **characterised in that** the device comprises at least one cover that closes the top of the device substantially fluid-tight in the passive position.

5. Device according to claim 4, **characterised in that** the connector is connected with the cover and is located inside the device in the passive position.

6. Device according to one of the above mentioned claims, **characterised in that** the device comprises at least one driver that presses the connector from the passive position in the direction of the active position.

7. Medical device arrangement for the treatment of human or animal bodies, with at least two medical devices, electrically and mechanically connected with
each other via an electrical connector arrangement, whereby the connector arrangement comprises a connector connected with the first device, and a complementary mating connector connected with the second device, **characterised in that** the first medical device is designed according to one of the above mentioned claims.

8. Device arrangement according to claim 7, **characterised in that** the first device is an ultrasound generator, and the second device is a HF generator.

9. Device arrangement according to claim 8, **characterised in that** the device arrangement is designed in such a way that a high frequency current signal as well as a signal for an ultrasound instrument are readable simultaneously at the ultrasound generator during operation.

## Revendications

1. Appareil médical pour le traitement du corps humain ou animal, comprenant au moins un connecteur électrique qui est réalisé de manière à pouvoir être connecté à un connecteur conjugué complémentaire d'un autre appareil médical et qui est disposé dans une position active au niveau du côté supérieur de l'appareil, de manière à faire saillie vers le haut, le connecteur étant réalisé de manière à pouvoir être déplacé de la position active dans une position passive, le connecteur, dans la position passive, étant éloigné du côté supérieur de l'appareil et le côté supérieur étant fermé de manière étanche aux liquides.

2. Appareil selon la revendication 1, **caractérisé en ce que** l'appareil présente au moins une unité de levage par le biais de laquelle le connecteur est connecté de manière déplaçable au reste de l'appareil.

3. Appareil selon la revendication 2, **caractérisé en ce que** l'unité de levage présente une articulation de poussée par le biais de laquelle le connecteur est réalisé de manière à pouvoir être déplacé essentiellement linéairement de la position active dans la position passive.

4. Appareil selon l'une quelconque des revendications précédentes, **caractérisé en ce que** l'appareil comprend au moins un moyen de recouvrement qui ferme le côté supérieur de l'appareil dans la position passive essentiellement de manière étanche aux liquides.

5. Appareil selon la revendication 4, **caractérisé en ce que** le connecteur est connecté au moyen de recouvrement et, dans la position passive, est disposé à l'intérieur de l'appareil.

6. Appareil selon l'une quelconque des revendications précédentes, **caractérisé en ce que** l'appareil présente au moins un moyen d'entraînement qui presse le connecteur de la position passive dans la direction de la position active.

7. Agencement d'appareils médicaux pour le traitement du corps humain ou animal, comprenant au moins deux appareils médicaux qui sont connectés l'un à l'autre électriquement et mécaniquement par le biais d'un agencement de connecteurs, l'agencement de connecteurs présentant un connecteur connecté au premier appareil et un connecteur conjugué connecté au deuxième appareil, réalisé de manière complémentaire au connecteur, **caractérisé en ce que** le premier appareil médical est réalisé selon l'une quelconque des revendications précédentes.

8. Agencement d'appareils selon la revendication 7, **caractérisé en ce que** le premier appareil est un générateur d'ultrasons et le deuxième appareil est un générateur de hautes fréquences.

9. Agencement d'appareils selon la revendication 8, **caractérisé en ce que** l'agencement d'appareils est réalisé de telle sorte que pendant le fonctionnement, un signal électrique haute fréquence ainsi qu'un signal pour un instrument à ultrasons puissent être simultanément prélevés au niveau du générateur d'ultrasons.
